# EUROPÄISCHE PATENTSCHRIFT

(11) **EP 0 904 051 B1**
(45) Veröffentlichungstag und Bekanntmachung des Hinweises auf die Patenterteilung: **22.10.2003**
(21) Anmeldenummer: 97921736.1
(22) Anmeldetag: 24.04.1997
(51) Int. Cl.: A61K 7/48

(54) **KOSMETISCHE ODER PHARMAZEUTISCHE ZUBEREITUNGEN MIT VERMINDERTEM KLEBRIGKEITSGEFÜHL**
COSMETIC OR PHARMACEUTICAL PREPARATIONS WITH A REDUCED FEELING OF STICKINESS
COMPOSITIONS COSMETIQUES OU PHARMACEUTIQUES A EFFET COLLANT DIMINUE

(30) Priorität: 17.05.1996 DE 19619837
(43) Veröffentlichungstag der Anmeldung: 31.03.1999
(73) Patentinhaber: Beiersdorf Aktiengesellschaft, 20245 Hamburg (DE); CERESTAR HOLDING B.V., NL-4551 LA Sas van Gent (NL)
(72) Erfinder: FÄNGER, Sabine, D-20255 Hamburg (DE); VON DER FECHT, Stephanie, D-22869 Schenefeld (DE); HAEST, Gertrudis, B-3000 Leuven (BE); SCHNEIDER, Günther, D-20253 Hamburg (DE)
(86) Internationale Anmeldenummer: EP9702091
(87) Internationale Veröffentlichungsnummer: WO97044009

(56) Entgegenhaltungen:
- EP-A- 0 309 353
- EP-A- 0 583 756
- WO-A-95/06458
- GB-A- 2 076 290
- US-A- 3 852 475
- CHEMICAL ABSTRACTS, vol. 124, no. 1, 1.Januar 1996 Columbus, Ohio, US; abstract no. 7401, TRUBIANO, PAOLO C.: "The role of specialty food starches in flavor encapsulation" XP002038184 & ACS SYMP. SER. (1995), 610(FLAVOR TECHNOLOGY), 244-53 CODEN: ACSMC8;ISSN: 0097-6156, 1995,

## Beschreibung

Die vorliegende Erfindung betrifft kosmetische oder pharmazeutische Zubereitungen mit vermindertem Klebrigkeitsgefühl, Verfahren zu ihrer Herstellung sowie die Verwendung von Wirkstoffen zur Herabminderung des Klebrigkeitsgefühles kosmetischer Zubereitungen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es femer, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

So wie man also bei den einfachen Emulsionen von W/O- oder O/W-Emulsionen spricht (Wasser-in-Oel oder Oel-in-Wasser), gibt es bei multiplen Emulsionen W/O/W-, O/W/O-, O/W/O/W-, W/O/W/O-Emulsionen und so fort.

Die Tröpfchendurchmesser der gewöhnlichen Emulsionen liegen im Bereich von ca 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulichweißgefärbt und undurchsichtig.

Mizellaren und molekularen Lösungen mit Partikeldurchmessem kleiner als ca. 10⁻² µm, ist vorbehalten, klar und transparent zu erscheinen.

Der Tröpfchendurchmesser von transparenten bzw. transluzenten Mikroemulsionen dagegen liegt im Bereich von etwa 10⁻² µm bis etwa 10⁻¹ µm. Solche Mikroemulsionen sind meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe feiner dispers vorliegen können als in der dispersen Phase von "Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

An sich ist die Verwendung der üblichen kosmetischen Emulgatoren unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt.

Emulgatorfreie kosmetische Zubereitungen, oft ausgestaltet als Lichtschutzpräparate, auf Basis sogenannter Hydrodispersionen sind seit einiger Zeit für den Verbraucher zugängig.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

W/O-Lipodispersionen, welche den Gegenstand der vorliegenden Erfindung darstellen, sind in umgekehrter Analogie emulgatorfreie feindisperse Zubereitungen vom Typ Wasser-in-Öl.

Übliche, und sich gerade in neuerer Zeit immer weiter verbreitende kosmetische und dermatologische Zubereitungsformen, welche emulgatorfrei aber auch emulgatorhaltig sein können, sind Gele. Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind femer auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind. Meistens sind Gele durchsichtig. In der kosmetischen bzw. pharmazeutischen Galenik zeichnen sich Gele in aller Regel durch halbfeste, oft fließfähige Konsistenz aus.

Auch kosmetische Stifte, insbesondere Lippenstifte, bevorzugt Lippenpflegestifte, aber auch desodorierende Stifte ("Deo-Sticks") sind gebräuchliche Zubereitungen.

Die Haut der Lippen besitzt nur eine äußerst dünne Homschicht. Schweißdrüsen sind auf den Lippen gar nicht, Talgdrüsen nur vereinzelt zu finden. Daher ist die Lippenhaut praktisch frei von Fett und neigt, besonders bei kaltem und trockenem Wetter, zum Austrocknen. Dabei können sich kleine Risse in der Haut bilden, und die Empfindlichkeit der Lippen gegenüber chemischen, physikalischen und mikrobiellen Einwirkungen (z.B. Nahrungsmittel, Sonnenlicht, Herpes-Simplex-Viren) steigt.

Dies zu verhindern ist die Aufgabe von Lippenpflegestiften. Diese Produkte enthalten meist zu einem hohen Anteil Wachse und Fettkomponenten, die nach dem Auftragen eine abdeckende Schicht über den Lippen ausbilden.

Technisch betrachtet sind fast alle Lippenstifte wasserfreie Fettmischungen aus festen oder halbfesten Wachsen und flüssigen Ölen, wobei die hochgereinigten Paraffinöle und -wachse die Lippenstiftgrundmasse darstellen.

Nach dem idealen Anforderungsprofil sollen sich Lippenstifte, aber auch andere kosmetische Stiftformulierungen, z.B. desodorierend wirkende Stifte (sogenannte Deo-Sticks) glatt und ohne großen Reibungswiderstand auftragen lassen. Ein Lippenstift insbesondere soll schon bei leichtem Andruck einen nicht schmierigen, stumpfen oder klebrigen, aber dennoch gut haftenden Fettfilm an die Lippen abgeben. Durch diesen Fettfilm sollen die Lippen dann glatt und geschmeidig gemacht werden.

Ein Stilmittel der dekorativen Kosmetik ist, die Lippenfarbe durch entsprechende Kosmetika auf den Typ der Person abzustimmen.

Produkte dieser Art sind beispielsweise dekorative Lippenstifte, in welche verschiedenste Farbpigmente eingearbeitet werden können. Diese Stifte enthalten zu hohen Anteilen Wachse und Fettkomponenten, die nach dem Auftragen eine abdeckende Lipidschicht über den Lippen ausbilden.

Insbesondere Zubereitungen zur kosmetischen oder therapeutischen Hautpflege enthalten als wesentliche Bestandteile Abmischungen aus Ölen bzw. öllöslichen Substanzen und Wasser bzw. wasserlöslichen Substanzen. Bestimmte Bestandteile der Wasserphase, z.B. Glycerin, aber auch der Ölphase, z.B.Tocopherylacetat wirken sich in höheren Konzentrationen negativ auf die sensorischen Eigenschaften der Zubereitungen aus. Oft äußert sich dies in einem gesteigerten Klebrigkeitsgefühl oder auch Schmierigkeitsgefühl bei der Anwendung entsprechender Zubereitungen, welche dann im Einzelfalle nicht vermarktungsfähig sein können, da sie vom Verbraucher nicht akzeptiert bzw. negativ beurteilt werden.

Es ist zwar bekannt, durch Hinzufügen bestimmter Substanzen, beispielsweise einiger ausgewählter Puderrohstoffe, insbesondere Talkum, dieses Klebrigkeitsgefühl oder auch Schmierigkeitsgefühl zu reduzieren. Davon abgesehen, daß dieses nur selten vollständig gelingt, wird durch einen solchen Zusatz auch die Viskosität des betreffenden Produktes verändert und die Stabilität verringert.

Aufgabe war daher, all diesen den Nachteilen des Standes der Technik Abhilfe zu schaffen. Insbesondere sollten Produkte mit verringerter Klebrigkeit bzw. Schmierigkeit zur Verfügung gestellt werden. Produkte auf dem Gebiete der pflegenden Kosmetik, der dekorativen Kosmetik und der pharmakologischen Galenik sollten gleichermaßen von den geschilderten Nachteilen des Standes der Technik befreit werden.

Weiterhin war es eine Aufgabe der Erfindung, kosmetische Grundlagen für kosmetische Zubereitungen zu entwickeln, die sich durch gute Hautverträglichkeit auszeichnen.

Femer war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

Erstaunlicherweise werden all diese Aufgaben gelöst durch kosmetische oder pharmazeutische Zubereitungen, dadurch gekennzeichnet, daß sie eine wirksame Konzentration an mit einem oder mehreren n-Octenylsuccinatresten veresterter hydrophiler Stärke, wie im Anspruch definiert, enthalten.

Erfindungsgemäß ist femer die Verwendung von mit einem oder mehreren n-Octenyl-succinatresten veresterter hydrophiler Stärke, wie im Anspruch 1 definiert, zur Verminderung der Klebrigkeit und/oder Schmierigkeit kosmetischer oder pharmazeutischer Zubereitungen.

Die erfindungsgemäß verwendeten Stärkederivate zeichnen sich aus durch eine Struktur

Stärke-Xₙ, wobei X den Rest 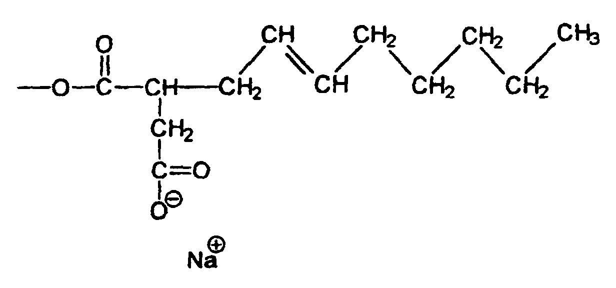 symbolisiert.

Erfindungsgemäß vorteilhaft zu verwendende Stärkederivate tragen offiziell noch keinen INCl-Namen (International Nomenclature Cosmetic Ingredient) dieser müßte die Bezeichnung "Starch Sodium Octenyl Succinate" tragen. Besonders vorteilhaft sind solche Produkte, welcher unter der Bezeichnung Amiogum®, insbesondere Amiogum®23 von der Geseltschaft Cerestar US verkauft werden.

Erfindungsgemäß kann der Gehalt an erfindungsgemäß verwendeten Stärkederivaten (im folgenden auch "erfindungsgemäß verwendeter Wirkstoff" genannt) in den kosmetischen oder topischen dermatologischen Zubereitungen 0,01 - 25 Gew.-%, bevorzugt 0,1 - 10 Gew.-%, insbesondere 0,2 - 5,0 Gew.-% betragen, bezogen auf das Gesamtgewicht der Zubereitungen.

Es hat sich überraschenderweise herausgestellt, daß der erfindungsgemäß verwendete Wirkstoff die der Erfindung zugrundeliegenden Aufgaben erfüllt.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Insbesondere kann der erfindungsgemäß verwendete Wirkstoff als Zusatzstoff in kosmetischen Desodorantien oder Antitranspirantien verwendet werden. Als desodorierend bzw. antitranspirierend wirksame Agentien können dann die üblichen, dem Fachmanne bekannten Substanzen verwendet werden. Beispielsweise kann durch Adstringentien - vorwiegend Aluminiumsalze wie Aluminiumhydroxychlorid (Aluchlorhydrat) - die Entstehung des Schweißes unterbunden werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Vorteilhaft sind beispielsweise Monocarbonsäureester des Di- bzw. Triglycerins. Aber auch andere antimikrobiell wirksame Stoffe sind geeignet.

Erfindungsgemäß ist sogar die Verwendung an sich nicht sonderlich milder desodorierender oder als Antitranspirantien wirkende Wirkstoffe möglich und gegebenenfalls vorteilhaft, da deren eventuelle erythemfördemde Wirkung vom erfindungsgemäß verwendeten Wirkstoff kompensiert werden kann.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthatten. So werden beispielsweise in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate. vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2.2'-Dihydroxy-4-methoxybenzophenon;
- Ester der Benzalmalonsäure, vorzugsweise 4-Methoxybenzalmalonsäuredi(2-ethylhexyt)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen kosmetischen und dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Es ist vorteilhaft, den Zubereitungen im Sinne der vorliegenden Erfindung weitere antiirritative oder antientzündliche Wirkstoffe zuzugeben, insbesondere Batylalkohol (α-Octadecylglycerylether), Selachylalkohol (α-9-Octadecenylglycerylether), Chimylalkohol (α-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyt- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Femer kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase femer einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind femer Mischungen aus Cyclomethicon und Isotridecylisononanoat, aus Cyclomethicon und 2-Ethylhexylisostearat.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester. Bevorzugt werden Lippenpflegestifte sowie Stiftformulierungen zur Körperdesodorierung.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Camaubawachs, Candelillawachs)

Als Treibmittel für aus Aerosolbehältem versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Liegen die kosmetischen oder dermatologischen Zubereitungen im Sinne der vorliegenden Erfindung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wir, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetischen und/oder dermatologischen Zubereitungen können auch Aerosole mit den üblicherweise dafür verwendeten Hilfsmitteln darstellen.

Eine kosmetische Zubereitung in Form einer Lotion im Sinne der vorliegenden Erfindung, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie die erfindungsgemäßen Wirkstoffkombinationen. Die Menge der verwendeten Polymeren liegt z.B. zwischen 0,1 und 10 Gew.-%, bevorzugt zwischen 0,1 und 3 Gew.-%.

Kosmetische Zubereitungen im Sinne der vorliegenden Erfindung zur Behandlung und Pflege der Haare, die den erfindungsgemäß verwendeten Wirkstoff enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Kosmetische Zubereitungen im Sinne der vorliegenden Erfindung zur Behandlung und Pflege der Haare können als Gele vorliegen, die neben einem wirksamen Gehalt am erfindungsgemäßen Wirkstoff und dafür üblicherweise verwendeten Lösungsmitteln. bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder - distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen.

### Beispiel 1 (W/O-Emulsion):

| | Gew.-% |
|---|---|
| PEG-7-hydriertes Ricinusöl | 4,00 |
| Wollwachsalkohol | 1,50 |
| Bienenwachs | 3,00 |
| Vaseline | 4,00 |
| Ozokerit | 4,00 |
| Paraffinöl, subliquidum | 10,00 |
| Glycerin | 15,00 |
| Octyl Methoxycinnamat | 2,50 |
| Methylbenzylidene Camphor | 2,50 |
| Tocopherolacetat | 1,00 |
| Amiogum®23 (Starch Sodium Octenyl Succinate) | 0,50 |
| Magnesiumsulfat 7 H₂O | 0,70 |
| Parfüm, Konservierungsmittel, | |
| Farbstoffe, Antioxidantien etc. | q.s. |
| Wasser | ad 100,00 |

### Beispiel 2 (W/O-Emulsion):

| | Gew.-% |
|---|---|
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Paraffinöl, subliquidum | 10,00 |
| Sorbit | 15,00 |
| Amiogum®23 (Starch Sodium Octenyl Succinate) | 0,50 |
| Magnesiumsulfat 7 H₂O | 0,70 |
| Parfüm, Konservierungsmittel, | |
| Farbstoffe, Antioxidantien etc. | q.s. |
| Wasser | ad 100,00 |

### Beispiel 3 (W/O-Emulsion):

| | Gew.-% |
|---|---|
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Paraffinöl, subliquidum | 10,00 |
| Harnstoff | 10,00 |
| Amiogum®23 (Starch Sodium Octenyl Succinate) | 0,50 |
| Magnesiumsulfat 7 H₂O | 0,70 |
| Milchsäure | 0.30 |
| Natriumiactat | 2,50 |
| Parfüm, Konservierungsmittel, | |
| Farbstoffe, Antioxidantien etc. | q.s. |
| Wasser | ad 100,00 |

### Beispiel 4 (W/O-Emulsion):

| | Gew.-% |
|---|---|
| PEG-7-hydriertes Ricinusöl | 4,00 |
| Wollwachsalkohol | 1,50 |
| Bienenwachs | 3,00 |
| Vaseline | 9,00 |
| Ozokerit | 4,00 |
| Paraffinöl, subliquidum | 10,00 |
| Glycerin | 15,00 |
| Tocopherolacetat | 1,00 |
| Amiogum®23 (Starch Sodium Octenyl Succinate) | 0,50 |
| Zinksulfat 7 H₂O | 0,70 |
| Glycin | 1.50 |
| Parfüm, Konservierungsmittel, NaOH | |
| Farbstoffe, Antioxidantien etc. | q.s. |
| Wasser | ad 100,00 |

### Beispiel 5 (W/O-Emulsion):

| | Gew.-% |
|---|---|
| Polyglyceryl-3 Dioleat | 3,50 |
| Ozokerit | 3,00 |
| Bienenwachs | 2,00 |
| Paraffinöl, subliquidum | 10,00 |
| Cetearyloctanoat | 10.00 |
| Serin | 0,50 |
| Sorbit | 9,00 |
| Glycerin | 9,00 |
| Amiogum® 23 (Starch Sodium Octenyl Succinate) | 0,50 |
| Magnesiumsulfat 7 H₂O | 0,70 |
| Parfüm, Konservierungsmittel, | |
| Farbstoffe, Antioxidantien etc. | q.s. |
| Wasser | ad 100,00 |

### Beispiel 6 (W/O-Emulsion):

| | Gew.-% |
|---|---|
| Laurylmethicone Copolyol | 1,50 |
| Cetylmethicone Copolyol | 0,50 |
| Paraffinöl, subliquidum | 10,00 |
| Cylomethicone | 2,00 |
| Dimethicone | 1,00 |
| Weizenkeimöl | 4,00 |
| Capric/Caprylic Triglyceride | 4,00 |
| Glycerin | 10,00 |
| Amiogum®23 (Starch Sodium Octenyl Succinate) | 0,25 |
| Natriumchlorid | 1,00 |
| Parfüm, Konservierungsmittel, | |
| Farbstoffe, Antioxidantien etc. | q.s. |
| Wasser | ad 100,00 |

### Beispiel 7 (O/W-Emulsion):

| | Gew.-% |
|---|---|
| Sorbitanmonostearat | 2,50 |
| Glycerinmonostearat | 1,00 |
| Vaseline | 0,50 |
| Paraffinöl, subliquidum | 11,00 |
| Octyldodecanol | 4,00 |
| Hydrierte Kokosfettsäureglyceride | 1,00 |
| Cyclomethicone | 1,00 |
| Carbomer | 0,15 |
| Glycerin | 10,00 |
| Tocopherylacetat | 1,00 |
| Amiogum® 23 (Starch Sodium Octenyl Succinate) | 0,50 |
| Parfüm, Konservierungsmittel, NaOH, | |
| Farbstoffe, Antioxidantien etc. | q.s. |
| Wasser | ad 100,00 |

### Beispiel 8 (O/W-Emulsion):

| | Gew.-% |
|---|---|
| Stearinsäure | 1,50 |
| Sorbitanmonostearat | 0,50 |
| Myristylalkokol | 1,50 |
| Glycerinmonostearat | 0,50 |
| Paraffinöl, subliquidum | 10,00 |
| Dimethicone | 1,00 |
| Octyldodecanol | 2,00 |
| Hydrierte Kokosfettsäureglyceride | 0,50 |
| Carbomer | 0,10 |
| Serin | 0.50 |
| Glycerin | 5,00 |
| Tocopherylacetat | 0,50 |
| Amiogum®23 (Starch Sodium Octenyl Succinate) | 0,50 |
| Parfüm, Konservierungsmittel, NaOH, | |
| Farbstoffe, Antioxidantien etc. | q.s. |
| Wasser | ad 100,00 |

### Beispiel 9 (O/W-Emulsion):

| | Gew.-% |
|---|---|
| Sorbitanmonostearat | 2,00 |
| Laurylmethicone Copolyol | 0,35 |
| Cetylmethicone Copolyol | 0,15 |
| Paraffinöl, subliquidum | 10,00 |
| Octyldodecanol | 4,00 |
| Hydrierte Kokosfettsäureglyceride | 1,00 |
| Cyclomethicone | 1,00 |
| Dimethicone | 1,00 |
| Carbomer | 0,15 |
| Glycerin | 10,00 |
| Tocopherylacetat | 1,00 |
| Amiogum®23 (Starch Sodium Octenyl Succinate) | 0,50 |
| Parfüm, Konservierungsmittel, NaOH | |
| Farbstoffe, Antioxidantien etc. | q.s. |
| Wasser | ad 100,00 |

### Beispiel 10 (O/W-Emulsion):

| | Gew.-% |
|---|---|
| Quatemium-5 | 3.50 |
| Paraffinöl, subliquidum | 10,00 |
| Cetearylalkohol | 2.00 |
| Hydrierte Kokosfettsäureglyceride | 0,50 |
| Dimethicone | 0.75 |
| Glycerin | 20,00 |
| Tocopherylacetat | 0,50 |
| Amiogum®23 (Starch Sodium Octenyl Succinate) | 0,50 |
| Parfüm, Konservierungsmittel, NaOH | |
| Farbstoffe, Antioxidantien etc. | q.s. |
| Wasser | ad 100,00 |

### Beispiel 11 (Hydrodispersionsgel):

| | Gew.-% |
|---|---|
| PEG-8 (Polythylenglycol 400) | 5.00 |
| Ethanol | 2,00 |
| Carbomer | 0,70 |
| Triglycerid, flüssig | 1,50 |
| Amiogum^{⊗}23 (Starch Sodium Octenyl Succinate) | 1,25 |
| Glycerin | 5,00 |
| Sorbit | 2,00 |
| Panthenol | 0,50 |
| Tocopherylacetat | 0,50 |
| Parfüm, Konservierungsmittel, NaOH, | q.s. |
| Farbstoffe, Antioxidantien etc. | |
| Wasser | ad 100,00 |

### Beispiel 12 (Hydrogel):

| | Gew.-% |
|---|---|
| PEG-8 (Polythylenglycol 400) | 5,00 |
| Ethanol | 2,00 |
| Carbomer | 0,70 |
| Amiogum®23 (Starch Sodium Octenyl Succinate) | 1,25 |
| Glycerin | 5,00 |
| Sorbit | 2,00 |
| Panthenol | 0,50 |
| Tocopherylacetat | 0.50 |
| Parfüm, Konservierungsmittel, NaOH, | q.s. |
| Farbstoffe, Antioxidantien etc. | |
| Wasser | ad 100,00 |

### Beispiel 13 (Lippenpflegestift):

| | Gew.-% |
|---|---|
| Caprylic/Capric Triglyceride | 27,00 |
| Octyldodecanol | 27,00 |
| Bienenwachs | 15,00 |
| Cetylpalmitate | 5,00 |
| Amiogum®23 (Starch Sodium Octenyl Succinate) | 0,25 |
| Squalan | 13,00 |
| Jojoba Öl | 10,00 |
| Camauba Wachs | 2,00 |
| Tocopherolacetat | 0,75 |
| Parfüm, Konservierungsmittel, Antioxidantien etc. | q.s. |

### Beispiel 14 (Emulsions-Lippenpflegestift):

| | Gew.-% |
|---|---|
| Caprylic/Capric Triglyceride | 30,00 |
| Octyldodecanol | 20,00 |
| Polyglyceryl-3 Dioleat | 3,50 |
| Bienenwachs | 12,50 |
| Amiogum®23 (Starch Sodium Octenyl Succinate) | 0,25 |
| Squalan | 11,00 |
| C20-40 Alkyl Stearate | 5,00 |
| Jojoba Öl | 10,00 |
| Camauba Wachs | 2,00 |
| Tocopherylacetat | 0,75 |
| Wasser | 5,00 |
| Parfüm, Konservierungsmittel, Antioxidantien etc. | q.s. |

### Beispiel 15 (Lippenpflegegel)

| | Gew.-% |
|---|---|
| Caprylic/Capric Triglyceride | 40,00 |
| Vaseline | 40,00 |
| Wollwachsalkohol | 1,00 |
| Bienenwachs | 3,00 |
| Amiogum®23 (Starch Sodium Octenyl Succinate) | 2,50 |
| Polyisobutene | 5,00 |
| Jojoba Öl | 8,00 |
| Tocopherylacetat | 0,50 |
| Parfüm, Konservierungsmittel, Antioxidantien etc. | q.s. |

### Beispiel 16 (Gesichtspuder, gepreßt):

| | |
|---|---|
| Magnesiumsilikat | 25,00 |
| Magnesiumstearat | 1,50 |
| Neylon-12 | 2,50 |
| Lauroyl Lysine | 1,00 |
| Kaolin | 10,00 |
| Magnesiumcarbonat | 5,00 |
| Amiogum®23 (Starch Sodium Octenyl Succinate) | 3,50 |
| Glimmer | 5,00 |
| Eisenoxide | 1,50 |
| Titandioxid | 2,50 |
| Isopropylisostearat | 2,50 |
| Paraffinöl, subliquidum | 2,50 |
| Talkum | ad 100,00 |
| Parfüm, Konservierungsmittel, Antioxidantien etc. | q.s. |

### Beispiel 17 (Emulsions-Make-up):

| | Gew.-% |
|---|---|
| Sorbitanmonostearat | 1,50 |
| Sorbitanmonooleat | 1,00 |
| Glycerinmonostearat | 1,00 |
| Paraffinöl, subliquidum | 7,00 |
| Octyldodecanol | 7,00 |
| Hydrierte Kokosfettsäureglyceride | 4,00 |
| Octyl Methoxycinnamat | 2,00 |
| Butyl Methoxydibenzoylmethane | 1,00 |
| Carbomer | 0,10 |
| Glycerin | 5,00 |
| 1,3 Butylenglycol | 2,00 |
| Tocopherolacetat | 1,00 |
| Amiogum® 23 (Starch Sodium Octenyl Succinate) | 2,50 |
| Magnesiumsilikat | 1,00 |
| Glimmer | 1,00 |
| Eisenoxide | 1,00 |
| Titandioxid | 2,50 |
| Talkum | 5,00 |
| Parfüm, Konservierungsmittel, NaOH, Farbstoffe, Antioxidantien etc. | q.s. |
| Wasser | ad 100,00 |

## Patentansprüche

1. Kosmetische oder pharmazeutische Zubereitungen, **dadurch gekennzeichnet, daß** sie eine wirksame Konzentration an einer mit einer oder mehreren n-Octenylsuccinatresten veresterter hydrophiler Stärke enthalten, welche sich durch eine Struktur
Stärke-Xₙ auszeichnet, wobei X den Rest 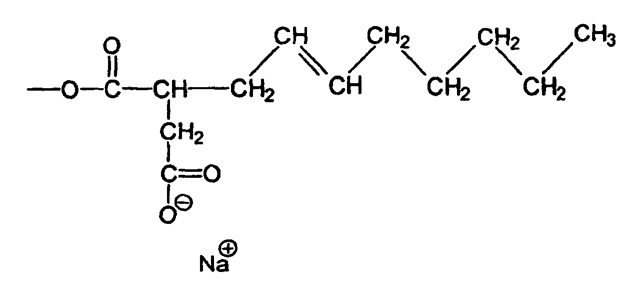 symbolisiert.

2. Zubereitungen nach Anspruch 1 , **dadurch gekennzeichnet, daß** der Gehalt an mit einem oder mehreren n-Octenylsuccinatresten veresterter hydrophiler Stärke in den kosmetischen oder topischen dermatologischen Zubereitungen 0,01 - 25 Gew.-%, bevorzugt 0,1 - 10 Gew.-%, insbesondere 0,2 - 5,0 Gew.-% beträgt, bezogen auf das Gesamtgewicht der Zubereitungen.

3. Verwendung von einer mit einer oder mehreren n-Octenylsuccinatresten veresterter hydrophiler Stärke, wie sie in Anspruch 1 oder 2 definiert ist, zur Verminderung der Klebrigkeit und/oder Schmierigkeit kosmetischer oder pharmazeutischer Zubereitungen.

## Claims

1. Cosmetic or pharmaceutical preparations, **characterized in that** they comprise an effective concentration of a hydrophilic starch esterified with one or more n-octenylsuccinate radicals, which starch is **characterized by** a structure
starch-Xₙ, where X symbolizes the radical

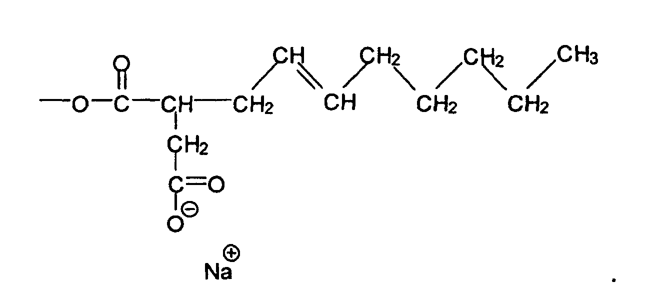

2. Preparations according to Claim 1, **characterized in that** the content of hydrophilic starch esterified with one or more n-octenylsuccinate radicals in the cosmetic or topical dermatological preparations is 0.01-25% by weight, preferably 0.1-10% by weight, in particular 0.2-5.0% by weight, based on the total weight of the preparations.

3. Use of a hydrophilic starch esterified with one or more n-octenylsuccinate radicals, as is defined in Claim 1 or 2, for reducing the stickiness and/or greasiness of cosmetic or pharmaceutical preparations.

## Revendications

1. Préparations cosmétiques ou pharmaceutiques, **caractérisées en ce qu'**elles contiennent une concentration efficace d'amidon hydrophile estérifié par un ou plusieurs radicaux n-octénylsuccinate, qui se distingue par une structure
amidon-Xₙ
X représentant le radical

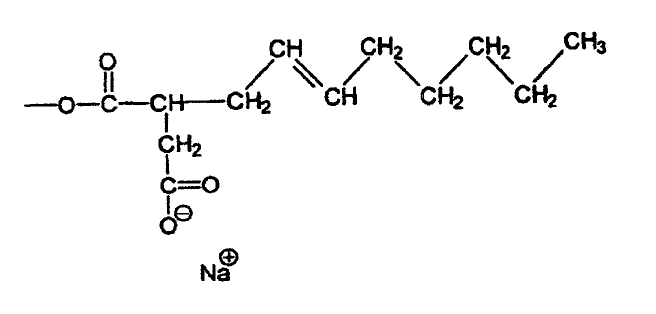

2. Préparations selon la revendication 1,
**caractérisées en ce que** la teneur en amidon hydrophile estérifié par un ou plusieurs radicaux n-octénylsuccinate des préparations cosmétiques ou dermatologiques topiques peut aller de 0,01 à 25 % en poids, de préférence de 0,1 à 10 % en poids, en particulier de 0,2 à 5,0 % en poids, par rapport au poids total des préparations.

3. Utilisation d'un amidon hydrophile estérifié par un ou plusieurs radicaux n-octénylsuccinate, tel que défini dans la revendication 1 ou 2, pour éviter l'état collant ou/et l'état graisseux de préparations cosmétiques ou pharmaceutiques.
